(19) Europäisches Patentamt
European Patent Office
Office européen des brevets

(11) Veröffentlichungsnummer: **0 010 179**
**B1**

(12) # EUROPÄISCHE PATENTSCHRIFT

(45) Veröffentlichungstag der Patentschrift: **19.05.82**

(21) Anmeldenummer: **79103563.7**

(22) Anmeldetag: **21.09.79**

(51) Int. Cl.³: **C 07 C 87/14,**
C 07 C 85/08,
C 07 C 85/12,
C 07 C 121/84

(54) **Verfahren zur Herstellung von 2.2-Dialkyl-pentan-1.5-diaminen und die Verbindungen N-(4-Cyano-2.2-diethyl-butyl)-4-cyano-2.2-diethyl-butyliden-imin und N-(4-Cyano-2-n-butyl-2-ethyl-butyl)-4-cyano-2-n-butyl-2-ethyl-butyliden-imin.**

(30) Priorität: **25.09.78 DE 2841585**

(43) Veröffentlichungstag der Anmeldung:
**30.04.80 Patentblatt 80/9**

(45) Bekanntmachung des Hinweises auf die Patenterteilung:
**19.05.82 Patentblatt 82/20**

(84) Benannte Vertragsstaaten:
**CH FR GB**

(56) Entgegenhaltungen:
**DE - A - 1 543 516**
**DE - A - 2 818 104**
**Chemical Abstracts Band 55, Nr. 23, 13. November 1961 Columbus, Ohio, USA R.H. HASEK et al. "Reaction of secondary and tertiary aldehydes with ammonia" Spalte 23328**
**Chemical Abstracts Band 71, Nr. 9, 1. September 1969 Columbus, Ohio, USA J. C. MILEO et al. "Monomers and polymers from 4-cyano-2,2-dimethyl-butyraldehyde. Synthesis of 1,5-diamino-2,2-dimethylpentane and polyamides with aromatic units" Seite 13, Spalte 2, Abstract Nr. 39537w**

(73) Patentinhaber: **Ruhrchemie Aktiengesellschaft**
**Bruchstrasse 219**
**D-4200 Oberhausen 13 (DE)**

(72) Erfinder: **Feichtinger, Hans, Dr. Dipl.-Chem**
**Gleiwitzer Strasse 6**
**D-4220 Dinslaken (DE)**
Erfinder: **Cornils, Boy, Dr. Dipl.-Chem.**
**Friedrich-Ebert-Strasse 45**
**D-4220 Dinslaken (DE)**
Erfinder: **Payer, Wolfgang. Dr. Dipl.-Chem.**
**Zedernweg 58**
**D-4230 Wesel 1 (DE)**

**EP 0 010 179 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 2,2-Dialkyl-pentan-1,5-diaminen und die Verbindungen N-(4-Cyano-2.2-diethyl-butyl)-4-cyano-2.2-diethyl-butyliden-imin und N-(4-Cyano-2-n-butyl-2-ethyl-butyl)-4-cyano-2-n-butyl-2-ethyl-butyliden-imin.

Die Erfindung betrifft ein Verfahren zur Herstellung von 2.2-Dialkyl-pentan-1.5-diaminen aus Aldehyden und Acrylnitril über 4-Cyano-2.2-dialkyl-butanale als Zwischenstufe.

4-Cyano-2.2-dialkyl-butanale sind aus $\alpha$-verzweigten Aldehyden und Acrylnitril leicht zugänglich. Aufgrund der gleichzeitigen Anwesenheit zweier funktioneller Gruppen im Molekül sind die genannten substituierten Butanale wertvolle Ausgangsverbindungen für weitere chemische Umsetzungen. Sowohl die Aldehyd- als auch die Nitrilgruppe sind allein oder gleichzeitig Umsetzungen verschiedener Art zugänglich. Genannt seien Reaktionen, die zur Bildung von Diaminen oder Dicarbonsäuren mit dem Neo-Kohlenstoffatom-Gerüst (2.2-Dialkylpentanstruktur) führen, die zur Herstellung von Polykondensaten mit speziellen Eigenschaften, wie Thermostabilität und Oxidationsbeständigkeit, geeignet sind.

Besonderes Interesse besitzen die Diamine, die zur Herstellung von Polyamiden, Polyharnstoffen und anderen polymeren Verbindungen eingesetzt werden können.

Die Herstellung von 2.2-Dialkyl-pentan-1.5-diaminen durch aminierende Hydrierung der entsprechenden Dialkylcyanobutanale ist von Mileo, Sillion, de Gaudemaris in C. R. Acad. Sci. Paris, Ser. C. *268* (1969), 1949 beschrieben. Die Umwandlung der Cyano-butanale in die entsprechenden Diamine gelingt nur unter drastischen Bedingungen, die Ausbeuten sind gering. So erhält man aus 4-Cyano-2.2-dialkyl-butanal bei 100° und einem $H_2/NH_3$-Gesamtdruck von 650 bar mit Äthanol als Lösungsmittel unter Verwendung von Raney-Kobalt als Katalysator nur 34%, unter Verwendung von Raney-Nickel als Katalysator nur 13% 2.2-Dimethyl-pentan-1.5-diamin. Hauptprodukte der Reaktion sind substituierte Piperidine bzw. Piperidone.

Es bestand daher die Aufgabe, ein Verfahren zu entwickeln, das die Herstellung der gewünschten 1.5-Diamine in einfacher Weise und mit guten Ausbeuten ermöglicht.

Die Herstellung von 2.2-Dialkyl-pentan-1.5-diaminen aus 4-Cyano-2.2-dialkyl-butanalen erfolgt erfindungsgemäß in der Weise, daß in einer ersten Stufe die 4-Cyano-2.2-dialkylbutanale bei 50 bis 250 bar und 80 bis 160°C in Gegenwart von Metallen der 8. Gruppe des Periodensystems als Katalysator mit Wasserstoff und Ammoniak gegebenenfalls in Gegenwart eines Lösungsmittels für das Cyano-butanal zum entsprechenden Azomethin umgesetzt und in einer zweiten Stufe das entstandene Azomethin bei 50 bis 500 bar und 50 bis 250°C in Gegenwart eines Kobaltkatalysators mit Wasserstoff und Ammoniak zur Reaktion gebracht wird.

Die als Ausgangsstoffe eingesetzten 4-Cyano-2.2-dialkylbutanale der allgemeinen Formel

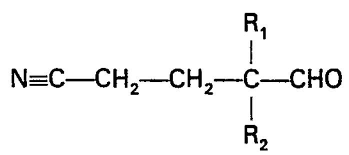

wobei $R_1$ und R gleiche oder verschiedene Alkylreste mit 1 bis 10 Kohlenstoffatomen bedeuten, sind aus $\alpha$-verzweigten Aldehyden und Acrylnitril leicht zugänglich. Ihre Herstellung ist, z.B. in der DE—PS 26 19 580 beschrieben.

Überraschenderweise hat sich herausgestellt, daß in zweistufiger Arbeitsweise die Cyanobutanale sich in höherer Ausbeute und selektiver in die entsprechenden Diamine überführen lassen, als bei einstufiger Reaktionsführung.

Die erste Stufe der Gesamtreaktion besteht in der Umsetzung des 4-Cyano-2.2-dialkyl-butanals mit Wasserstoff und Ammoniak. Die Reaktion erfolgt bei Drucken zwischen 50 bis 250 bar und bei Temperaturen von 80 bis 160°C. Als Katalysatoren werden Metalle der 8. Gruppe des Periodensystems eingesetzt, die als solche z.B. in feinverteilter Form, wie Raney-Nickel oder Raney-Kobalt oder in Form von Trägerkatalysatoren zum Einsatz gelangen. Besonders geeignet sind Kobalt-, Nickel-, Ruthenium-, Rhodium-, Palladium- oder Platin-Katalysatoren. Die Edelmetall-Trägerkatalysatoren enthalten üblicherweise 1 bis 10 Gew.% Metall, bezogen auf das Gesamtgewicht des Katalysators. Bei Nickel- oder Kobalt-Trägerkatalysatoren beträgt der Nickel- beziehungsweise Kobaltanteil 20 bis 80 Gew.%, bezogen auf das Gesamtgewicht des Katalysators. Als Träger kommen insbesondere Aluminiumoxid, Kieselsäure, künstliche oder natürliche Aluminiumsilikate, Kieselgur, Aktivkohle, Spinelle und Magnesiumsilikate in Betracht.

Die Reaktionstemperatur richtet sich nach der Art des eingesetzten Katalysators. Kobalt und Nickel erfordern im allgemeinen höhere Temperaturen — 110° bis 150°C — als die Edelmetallkatalysatoren, die schon bei Temperaturen von etwa 100°C hohe Wirksamkeit besitzen.

Auch der Reaktionsdruck ist von der Art des verwendeten Katalysators abhängig. Edelmetallkatalysatoren sind bereits in niederen Druckbereichen, d.h. bei etwa 60 bis 100 bar aktiv, während Kobalt und Nickelkatalysatoren üblicherweise Drücke von 200 bis 250 bar erfordern. Der gewünschte Druckbereich wird durch kontinuierliche Zuspeisung von Wasserstoff in dem Maße, wie er durch die Reaktion verbraucht wird, aufrecht erhalten.

Die Verwendung eines Lösungsmittels für das Cyanobutanal ist zweckmäßig. Als Lösungsmittel können niedere primäre Alkohole wie Äthanol, Anwendung finden. Die Menge des Lösungsmittels ist nicht kritisch und in weiten Grenzen variabel.

Gegenüber dem Aldehyd wird Ammoniak in großem Überschuß eingesetzt. Je Mol Aldehyd sollen mindestens 5 Mole Ammoniak zugegen sein. Die obere Grenze liegt bei einem Molverhältnis Aldehyd : Ammoniak von 1 : 50.

Als Produkt dieser ersten Reaktionsstufe entstehen nach der folgenden Reaktionsgleichung

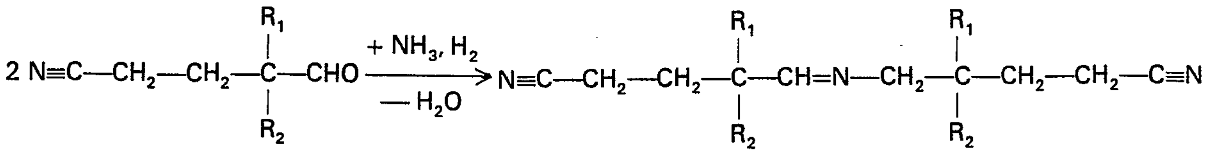


Azomethine. In der vorstehenden Gleichung haben die Substituenten $R_1$ und $R_2$ die oben angegebene Bedeutung.

Die Herstellung eines Azomethins der vorstehend angegebenen Struktur ist bereits Gegenstand einer Veröffentlichung von R. A. Hasek et al "Reaktion of Secondary and Tertiary Aldehydes with Ammonia" in J.Org.Chem. Band 26, 1961, Seiten 1822 bis 1825. Durch Umsetzung von 4-Cyano-2.2-dimethyl-butanal mit Ammoniak gelangen die Autoren zu dem entsprechenden Trialkyliden-diamin, das in einer zweiten Reaktionsstufe thermisch zu dem entsprechenden Azomethin gespalten wird. Gleichzeitig entsteht dabei je Mol Azomethin ein Mol Nitril. Abgesehen davon, daß diese Reaktion zweistufig ist, wobei die Bildung des Trialkyliden-diamins mit einer Ausbeute von 68% und die des Azomethins mit einer Ausbeute von 77% erfolgt, geht ein Drittel des eingesetzten Aldehyds für die Bildung des Azomethins verloren, weil — wie bereits gesagt — Nitril als Nebenprodukt entsteht.

In der zweiten Stufe werden die Azomethine aminierend hydriert. Diese Reaktion vollzicht sich ebenfalls unter Druck, nämlich bei 50 bis 500 bar und bei Temperaturen von 50 bis 250°C. Als Katalysator setzt man Kobalt ein, das in Form des feinverteilten Metalls, wie Raney-Kobalt oder in Form von Trägerkatalysatoren Anwendung finden kann. Die Trägerkatalysatoren enthalten 20 bis 80 Gew.% Kobalt, bezogen auf den Gesamtkatalysator. Als Träger kommen Aluminiumoxid, Kieselsäure, künstliche und natürliche Aluminiumsilikate, Kieselgur, Magnesiumsilikate, Spinelle und Aktivkohle in Betracht.

Das molare Verhältnis von Azomethin : Ammoniak wird auf 1 : 10 bis 50 Mol eingestellt. Wasserstoff wird dem Reaktionssystem in dem Maße zugeführt, daß sich der Druck auf den oben genannten Bereich einstellt.

Die in der zweiten Reaktionsstufe ablaufende Reaktion wird durch die folgende Gleichung wiedergegeben ($R_1$ und $R_2$ haben die oben angegebene Bedeutung)

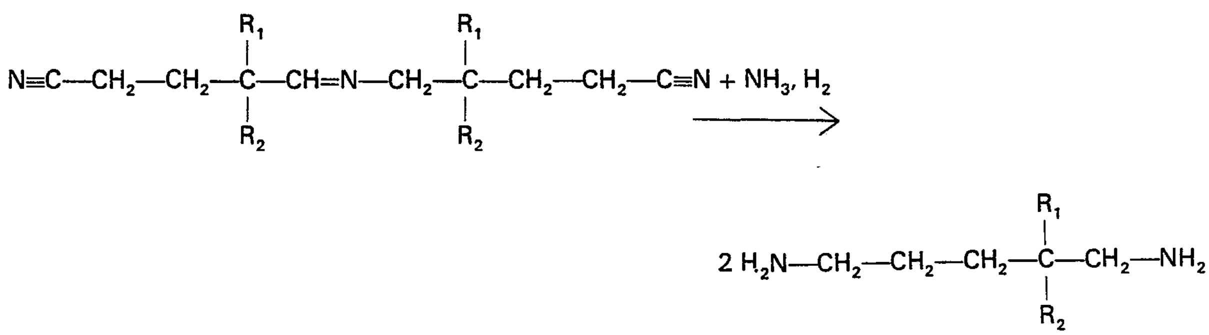

Beide Stufen können diskontinuierlich oder kontinuierlich in konventionellen Reaktoren, wie Autoklav oder Strömungsrohr durchgeführt werden.

Für die aminierende Hydrierung in der zweiten Reaktionsstufe ist es nicht erforderlich, das in der ersten Reaktionsstufe erhaltene Azomethin einem besonderen Reinigungsprozeß zu unterwerfen. Ohne daß die Diamin-Ausbeute beeinträchtigt wird, kann das Rohprodukt der ersten Reaktionsstufe weiter verarbeitet werden.

Nach dem erfindungsgemäßen Verfahren sind die neuen Verbindungen

1) N-(4-Cyano-2.2-diethyl-butyl)-4-cyano-2.2-diethylbutyliden-imin

2) N-(4-Cyano-2-n-butyl-2-ethyl-butyl)-4-cyano-2-n-butyl-2-ethyl-butyliden-imin

darstellbar.

Die Erfindung wird in den folgenden Beispielen näher erläutert.

Beispiel 1

In einem 2,8 1-Stahlautoklaven erhitzt man 500 g (4,0 Mol) 4-Cyano-2.2-dimethyl-butanal in Gegenwart von 25 g eines Pd-Trägerkatalysators (5 g Pd auf 95 g Aktivkohle) zusammen mit 700 g (41,0 Mol) flüssigem Ammoniak, hydriert mit Wasserstoff, der in einer solchen Menge vorliegt, daß sich bei 100°C ein Gesamtdruck von 80 bar einstellt. Die $H_2$-Aufnahme ist nach 3 Stunden beendet. Der flüssige Autoklaveninhalt wird vom Katalysator abfiltriert, es resultieren 440 g eines hellen Rohproduktes, das nach gaschromatographischer Analyse 82,1 Gew.% N-(4-Cyano-2.2-dimethyl-butyl)-4-cyano-2.2-dimethyl-butyliden-imin, bezogen auf die organische Phase enthält. Die Vakuumdestillation ergibt 320 g $C_{14}$-Azomethindinitril vom $Sdp_{0,5}$ 170°C. Die Reinheit beträgt nach gaschromatographischer Bestimmung 97,3%.

IR-Spektrum C≡N 2248 $cm^{-1}$
C=N 1670 $cm^{-1}$

n $n_D^{20}$ 1,4655

Beispiel 2

Entsprechend Beispiel 1 erhitzt man in einem 1 1-Stahlautoklaven 100 g (0,65 Mol) 4-Cyano-2.2-diethyl-butanal-gelöst in 200 ml Ethanol — in Gegenwart von 10 g eines Co-Trägerkatalysators (100 g enthalten etwa 40 g Co, Rest Kieselgur) zusammen mit 200 g (12 Mol) flüssigem Ammoniak. Anschließend leitet man soviel Wasserstoff ein, daß sich ein Gesamtdruck von 250 bar einstellt. Der während der Reaktion verbrauchte Wasserstoff wird laufend ergänzt Die Hydriertemperatur beträgt 110°C, die Versuchsdauer 2 Stunden.

Das nach der aminierenden Hydrierung erhaltene und vom Katalysator abfiltrierte Produkt enthält nach gaschromatographischer Bestimmung 49,9% (N-(4-Cyano-2.2-diethyl-butyl)-4-cyano-2.2-diethyl-butyliden-imin neben 26,3% Ausgangsverbindung, bezogen auf die organische Phase. Die anschließende Vakuumdestillation ergibt 45,2 g $C_{18}$-Azomethindinitril vom $Sdp_{0,1}$ 168°C. $n_D^{20}$ 1,4728. Die Reinheit beträgt nach gaschromatographischer Bestimmung 96,4%.

IR-Spektrum C≡N 2247 $cm^{-1}$
C=N 1665 $cm^{-1}$

Beispiel 3

Analog zu den Beispielen 1 und 2 erhitzt man 100 g (0,55 Mol) 4-Cyano-2-n-butyl-2-ethyl-butanal in Gegenwart von 10 g eines Ni-Trägerkatalysators (100 g enthalten etwa 55 g Ni, Rest Kieselgur) zusammen mit 150 g (8,8 Mol) flüssigem Ammoniak in einem 0,5 1-Stahlautoklaven. Der Gesamtdruck beträgt 250 bar, die Versuchstemperatur 140°C, die Hydrierzeit 3 Stunden. Der Autoklaveninhalt wird von Katalysator abfiltriert. Das Rohfiltrat enthält nach gaschromatographischer Analyse 61% N-(4-Cyano-2-n-butyl-2-ethyl-butyl)-4-cyano-2-n-butyl-2-ethyl-butyliden-imin, bezogen auf die organische Phase. Die Vakuumdestillation ergibt 55,2 g $C_{12}$-Azomethindinitril vom $Sdp_{0,3}$ 185°; $n_D^{20}$ 1,4798. Die Reinheit beträgt nach gaschromatographischer Bestimmung 98,9%.

IR-Spektrum: C≡N 2242 $cm^{-1}$
C=N 1660 $cm^{-1}$

Beispiel 4

100 g (0,43 Mol) N-(4-Cyano-2.2-dimethyl-butyl)-4-cyano-2.2-dimethylbutyliden-imin werden nach Beispiel 1 zusammen mit 150 g (8,8 Mol) flüssigem Ammoniak, 10 g eines Co-Trägerkatalysators (100 g enthalten etwa 40 g Co, Rest Kieselgur) und unter einem Gesamtdruck von 250 bar, der durch Ergänzen des verbrauchten Wasserstoffs aufrecht erhalten wird, auf 100°C erhitzt. Die Hydrierung ist nach 7,5 Stunden beendet. Man filtriert vom Katalysator ab, das flüssige Rohprodukt weist nach gaschromatographischer Analyse einen Gehalt von 65,2% 2.2-Dimethyl-pentan-1.5-diamin auf. Durch Destillation wird das Neo-$C_7$-diamin vom $Sdp_{14}$ 88—89 und einem Brechungsindex von $n_D^{20}$ 1,4618 in einer Ausbeute von 65,2% isoliert.

**Patentansprüche**

1. Verfahren zur Herstellung von 2.2-Dialkyl-pentan-1.5-diaminen, wobei die Alkylreste gleich oder verschieden sind und jeweils 1 bis 10 kohlenstoff- atome enthalten, aus 4-Cyano-2.2-dialkyl-butanalen, dadurch gekennzeichnet, daß

- in einer ersten Stufe die 4-Cyano-2.2-dialkyl-butanale
- bei 50 bis 250 bar und 80 bis 160°C
- in Gegenwart von Metallen der 8. Gruppe des Periodensystems als Katalysator
- mit Wasserstoff und Ammoniak

- gegebenenfalls in Gegenwart eines Lösungsmittels für das Cyanobutanal zum entsprechenden Azomethin umgesetzt und
- in einer zweiten Stufe das entstandene Azomethin bei 50 bis 500 bar und 50 bis 250°C
- in Gegenwart eines Kobaltkatalysators mit Wasserstoff und Ammoniak zur Reaktion gebracht wird.

2. Verfahren nach Anspruch 1, dadurch gekennzeichnet, daß in der ersten Stufe Kobalt-, Nickel-, Ruthenium-, Rhodium-, Palladium- oder Platin-Katalysatoren eingesetzt werden.

3. Die chemische Verbindung N-(4-Cyano-2.2-diethyl-butyl)-4-cyano-2.2-diethyl-butyliden-imin.

4. Die chemische Verbindung N-(4-Cyano-2-n-butyl-2-ethyl-butyl)-4-cyano-2-n-butyl-2-ethyl-butyliden-imin.

**Revendications**

1. Procédé de préparation de 2,2-dialkyl-pentane-1,5-diamines dans lesquelles les groupes alkyle, identiques ou différents, contiennent chacun de 1 à 10 atomes de carbone, à partir de 4-cyano-2,2-dialkyl-butanals, caractérisé en ce que:
- dans un premier stade, on fait réagir les 4-cyano-2,2-dialkyl-butanals
- sous une pression de 50 à 250 bars et à une température de 80 à 160°C
- en présence de métaux du huitième groupe de la Classification Périodique servant de catalyseurs,
- avec l'hydrogène et l'ammoniac,
- éventuellement en présence d'un solvant du cyanobutanal, la réaction donnant l'azométhine correspondante et
- dans un deuxième stade, on fait réagir l'azométhine obtenue sous une pression de 50 à 500 bars et à une température de 50 à 250°C
- en présence d'un catalyseur au cobalt avec l'hydrogène et l'ammoniac.

2. Procédé selon la revendication 1, caractérisé en ce que, au premier stade, on utilise des catalyseurs au cobalt, au nickel, au ruthénium, au rhodium, au palladium ou au platine.

3. Le composé chimique N-(4-cyano-2,2-diéthyl-butyl)-4-cyano-2,2-diéthyl-butylidène-imine.

4. Le composé chimique N-(4-cyano-2-n-butyl-2-éthyl-butyl)-4-cyano-2-n-butyl-2-éthyl-butylidène-imine.

**Claims**

1. Process for the manufacture of 2,2-dialkyl-pentane-1,5-diamines from 4-cyano-2,2-dialkyl-butanals, whereby the alkyl residues are identical or dissimilar and contain 1 to 10 carbon atoms, characterized in that
- during the first step conversion of the 4-cyano-2,2-dialkyl-butanals
- at 50 to 250 bar and 80 to 160°C
- in the presence of metals of the 8th group of the periodic table as catalyst
- with hydrogen and ammonia
- if necessary in the presence of a solvent for the cyanobutanal to the corresponding azomethine takes place, and
- in a second step reaction of the azomethine thus produced is induced
- at 50 to 500 bar and 50 to 250°C
- with hydrogen and ammonia in the presence of a cobalt catalyst.

2. Process in accordance with Claim 1, characterized in that in the first step cobalt, nickel, ruthenium, rhodium, palladium or platinum catalysts are used.

3. The chemical compound N-(4-cyano-2,2-diethyl-butyl)-4-cyano-2,2-diethyl-butylidene-imine.

4. The chemical compound N-(4-cyano-2-n-butyl-2-ethyl-butyl)-4-cyano-2-n-butyl-2-ethyl-butylidene-imine.